# EUROPEAN PATENT APPLICATION

(11) **EP 1 354 963 A1**
(43) Date of publication of application: **22.10.2003**
(21) Application number: 03008646.6
(22) Date of filing: 15.04.2003
(51) Int. Cl.: C12Q 1/68

(54) **Method for determining the prognosis of ovarian cancer**

(30) Priority: 15.04.2002 US 372626 P; 14.04.2003 US
(71) Applicant: HEALTH RESEARCH, INC., Buffalo New York 14263 (US)
(72) Inventor: Sood, Ashwani K., Williamsville,New York 14221 (US); Ghadersohi, Ali, Tonawanda, New York 14223 (US); Odunsi, Kunle, Williamsville, New York 14221 (US)
(74) Representative: Weber, Dieter, Dr.

(57) **Abstract**

The use of PDEF specific primer to determine prognosis of ovarian cancer characterized in that:
a sample of ovarian tissue cells to be tested is obtained;
mRNA is isolated from the sample;
the mRNA is treated with deoxyribonuclease;
cDNA's are generated from the treated mRNA by reverse transcription polymerase chain reaction (RT-PCR) using reverse transcriptase and PDEF specific primers are used to obtain PCR amplified product; and
the amplified product is analyzed to determine PDEF expression and the expression is compared with normal expression as an indication of prognosis of ovarian cancer.

## Description

This Application claims priority from U.S. Provisional Application No. 60/372,626 filed April 15, 2002.

### Background of the Invention

This invention relates to a method for determining prognosis for ovarian cancer. The invention more particularly relates to a method for determining the prognosis for ovarian cancer using an ovarian cancer marker.

Traditional methods for tumor detection have revolved around symptomatic analysis; palpation, where possible; sample excision followed by histological analysis; and where the tumor is large enough, x-ray, MRI, nuclear, thermal, and sonic imaging. All of the above methods of detection have associated problems for detection and have little if any probative value for prognosis, at least until late stage cancer.

Symptomatic analysis is a method that is only useful in relatively late stage tumor growth where the tumor has become sufficiently large to become visible, to become palpable or to interfere with one or more body functions. In addition, symptomatic analysis is usually not sufficient in itself to form a firm diagnosis since most symptoms can be caused by factors other than tumors, especially at early stages of tumor growth. Until late stage cancer, symptomatic analysis provides little assistance in determining how well a cancer patient will respond to therapeutic treatment and gives little assistance in predicting survival time.

Excision followed by histological analysis has been one of the most reliable methods of detection as related to samples taken from suspected tumor sites. Unfortunately, reliance must be had upon relatively subjective decisions based upon an experts microscopic evaluation of tissue structure. Further, unless routine samples are taken, which often requires uncomfortable and invasive techniques, samples are not taken until tumor growth has proceeded to a palpable or symptomatic size. Again, until late stage cancer, histological analysis provides little that helps in predicting survival time or treatment efficacy.

Imaging techniques also have associated disadvantages. Again, imaging techniques are not effective until tumor growth has proceeded to a visible size. Such techniques have further disadvantages. X-rays are not particularly good at imaging soft tissue and tumors are usually not visible until they are quite large. X-rays of course have the additional disadvantage of being ionizing radiation that causes tissue damage and increases cancer risks. MRI imaging requires an extended period of time in a confined space, requires exposure to high magnetic fields having risks yet to be determined and while much better than x-rays at imaging soft tissue, still do not clearly define tumors until of normally visible size. MRI imaging remains quite costly due to time involved and due to huge costs of machinery. Nuclear imaging, in addition to resulting undesirable radiation exposure, requires radioisotope uptake by a tumor or tissue around it, is time consuming and expensive and usually is incapable of detecting small tumors. Sonic imaging, to this point, has poor definition and is not capable of detecting small tumors and will not penetrate hard structures such as bone. Thermal imaging, e.g. mammography, can only be used close to the epidermal surface, has poor definition and is usually unreliable in the absence of a prior thermal image of the same structure to use as a baseline. Thermal imaging also has poor definition and misses many small tumors. As with the other detection methods discussed, this method helps very little with accurately predicting length of survival.

Recently, tumor specific markers have been used to detect the presence or absence of particular tumor types. One well known test being routinely employed is known as the "PSA" test. The test uses an antibody for prostate specific antigen to evaluate likelihood for the presence of prostate cancer. The test is non-invasive and does not often give false negatives; however false positives can run over 50% due to the fact that normal prostate tissue and non-cancerous abherent prostate tissue can produce the antigen in sufficient quantity to cloud the test results. The problem with false positives in antigenic cancer tests has been so significant that it has prevented clinical use of many antigen determinate tests for various cancers including cancers of lung, colon and ovaries. It has for example been known that certain ovarian tumor markers, e.g. Mucin-1, MUC-16 (CA-125), folate binding protein, and Her-2/neu, exist but unfortunately these markers are insufficiently expressed in ovarian tumor tissue or are expressed to a greater or lesser extent by normal tissue , e.g. choroid plexus, kidney, thyroid and lung, creating a masking effect that results in undesirable false positives. The test has not given reliable information concerning prognosis.

Published U.S. Patent Application 2001/0010934 is a primarily prophetic disclosure that indicates that prostate derived Ets factor (PDEF) is expressed in prostate epithelium and, without any support and contrary to its own examples, makes the statement that PDEF is expressed to a lesser extent in salivary glands, trachea, ovaries, and mammary tissues. The examples in the above published patent application in fact support no expression in ovaries at all and the only example in the patent publication (paragraph 0434) testing ovarian cancer showed no ovarian cancer expression.

To the present time there has been no marker that reliably permits a test permitting prognosis for ovarian cancer. It would be desirable to have such a predictive test to help determine a treatment plan and to permit a patient to make informed decisions with respect to personal courses of action.

### Brief Description of the Invention

In accordance with the invention a test method is provided that can provide an indication of good prognosis for the tumors detected. More specifically, it has been found that when PDEF is expressed by mRNA within a tissue sample, it is an indication of both the presence of ovarian cancer and that the prognosis for the cancer is favorable.

The use of PDEF specific primer to determine prognosis of ovarian cancer characterized in that:
a sample of ovarian tissue cells to be tested is obtained;
mRNA is isolated from the sample;
the mRNA is treated with deoxyribonuclease;
cDNA's are generated from the treated mRNA by reverse transcription polymerase chain reaction (RT-PCR) using reverse transcriptase and PDEF specific primers are used to obtain PCR amplified product; and
the amplified product is analyzed to determine PDEF expression and the expression is compared with normal expression as an indication of prognosis of ovarian cancer.

### Brief Description of the Drawings

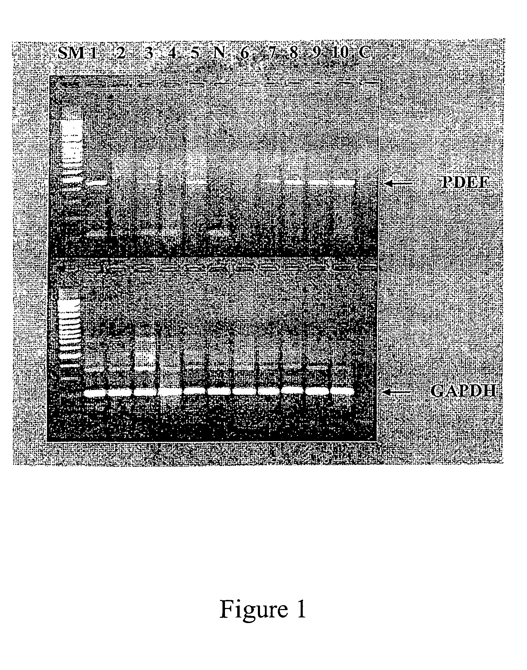

Figure 1 is a photograph of a gel showing expression of PDEF in 10 ovarian tumors and one normal ovarian tissue as obtained by RT/PCR assay. A one step RT/PCR assay was performed with 0.5 µg of RNA from each tissue analyzed on 1.8% agarose gel. The upper panel shows PDEF specific PCR and the lower panel shows GAPDH specific PCR. Lanes numbered 1 to 5 and 7 to 11 show PCR results from ovarian tumors and lane N shows PCR with RNA from normal ovary. The left lane marked SM contains 100 bp ladder markers and the right lane marked C is control PCR without RNA. Tumors 1,3,5,8,9,10 and 11 are positive for PDEF expression. In contrast, tumors 2, 4 and 7 are negative for PDEF expression as are the normal ovary sample and control.

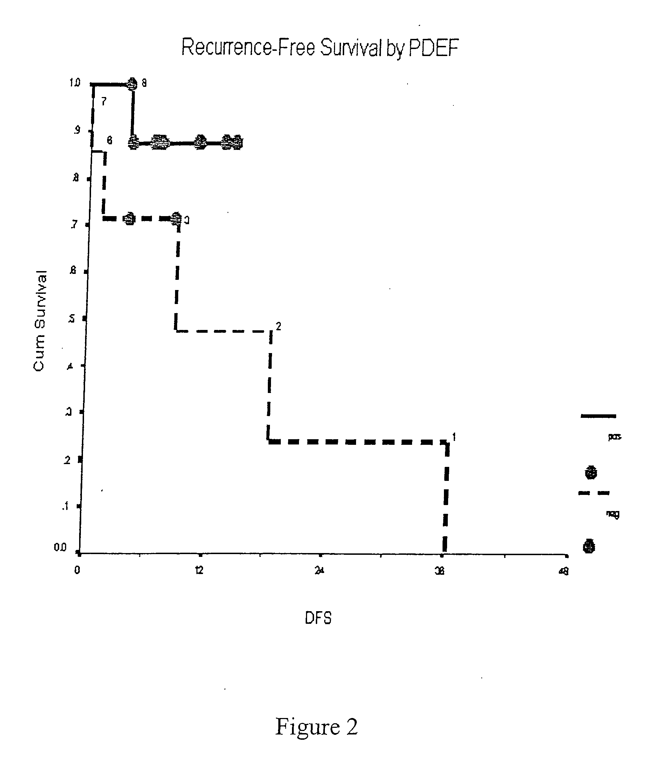

Figure 2 is a graph showing cumulative survival versus time in months for individuals having ovarian cancer that are PDEF negative (lower curve) and individuals having ovarian cancer that are PDEF positive (upper curve). The upper curve is for data from seven PDEF positive patients and the lower curve is from data from nine PDEF positive patients. Survival time is measured from the date of testing for PDEF. Sharp drops in the curves indicate the loss of a patient. The large dots on the curves represent patients still surviving and survival time at the time the curve was drawn.

### Detailed Description of the Invention

In accordance with the invention, Digital Differential Display (DDD) developed by the National Center for Biotechnology Information (NCBI), in association with the United States National Library of Medicine and the United States National Institute of Health, was used in the NCBI Unigene data base to determine that prostate epithelium derived Ets transcription factor (PDEF) is frequently over represented in ovarian carcinomas in cDNA libraries in comparison with those of various normal tissues.

To further test this observation, one-step reverse transcription polymerase chain reaction (RT-PCR) was performed upon RNA from various normal and serous epithelial ovarian tumor tissues using a partial PDEF sequence as a primer, followed by detection of amplified PDEF present, if any. Normal tissue RNA's were obtained from Clontech (Palo Alto, California) or were isolated from tissues obtained from the Tissue Procurement Facility of the Roswell Park Cancer Institute (RPCI), Buffalo, NY. A description of the normal tissues and tumors for which RNA was tested is given in Table 1.

More specifically, in the case of RNAs isolated from RPCI tissues, total RNA was obtained from tissue samples. Standard procedures may be used to isolate the RNA, e.g. the cells are homogenized and centrifuged to obtain RNA rich organelles. The isolated fractions may then be treated with a surfactant such as sodium dodecylsulfate (SDS) to release nucleic acids. The mixture may then be mixed with phenol or chloroform causing precipitation of proteins followed by centrifuging to leave DNA and RNA in solution in the upper aqueous phase that is separated. The process may be repeated until no further protein can be removed. The solution may then be treated with cold ethanol that causes the RNA to precipitate. The supernatant can then be removed, the RNA resuspended and treated with DNase to destroy any remaining DNA to obtain cellular RNA. In the practice of the present examples, for convenience a TriReagent™ kit obtained from Molecular Research Center, Inc., Cincinnati, Ohio was used to isolate RNA following the manufacturers instructions. Quality of isolated RNA was checked by electrophoresis on 1% agarose gels. Intact strong bands for 18S and 28S rRNAs without significant diffuse staining for low molecular weight RNAs was used as the criterion for assessing quality of the isolated RNAs. Based upon this test, the quality of isolated RNA's isolated from RPCI tissues favorably to the human tissue RNAs obtained commercially.

Suitable primers for cDNA's that appear in ovarian tumor tissues but not in normal ovarian tissues, as found by DDD as discussed above, were made by primer making methods known to those skilled in the art, e.g. as described on page 23, paragraphs 0251 and 0252 of U.S. Patent Application Publication US 2001/0010934 A1. In particular sense and anti-sense primers using a sequence selected from PDEF were made having the following sequences: 5’-GACCAGTGAGGAGAGCTGGACCGA-3’ and

5’-TGACCTTGGGCTCTGGAAGGTCAG-3’

One step RT/PCR mRNA amplification was performed with 0.5 µg of RNA using a suitable reverse transcriptase, e.g. M-MLV reverse transcriptase. More particularly the RT/PCR was performed using RNA from various normal and tumor tissues with a one-step RT-PCR kit obtained from Clontech and following the manufactures instructions. A 452 bp long PDEF specific PCR product was amplified using the PDEF specific primers above described. Glyceraldehyde-3-phosphodehydrogenase (GAPDH)-specific and 13-actin-specific PCR amplification were used as controls. Amplification was performed in a programmable thermal controller using a 60 minute incubation at 50°C for reverse transcription and 5 minutes at 95°C for enzyme deactivation followed by 25 to 40 cycles of PCR. Each PCR cycle consisted of a 30 second denaturization step at 94°C followed by a 30 second annealing step at 66°C and a one minute thirty second extension step at 72°C. After the last cycle, the final extension step was at 72°C for 10 minutes. Except for the use of different primers, the RT-PCR method was similar to the method described on pages 18 and 19, paragraph 200 of U.S. Patent Application Publication US 2001/0010934 A1.

The amplified product was detected using any suitable known method, e.g. SDS-polyacrylamide gel electrophoresis (SDS-PAGE). Other methods may also be used e.g. radioimmunoassay or ELISA. Such other methods may however be more sensitive than required due to the relatively large quantity of product to be analyzed in a PCR sample.

The χ² test was used to analyze the relationship between PDEF expression and clinical outcome. Survival distributions were calculated by the Kaplan-Meier method and statistical significance was calculated with the log-rank p test.

PDEF was detected at a 673 fold higher frequency in the cDNA from ovarian tumors in comparison to those from normal ovarian tissue (none determined). PDEF was virtually undetectable in other normal human tissues. RT-PCR expression analysis showed PDEF to be over expressed in 13 out of 22 (59%) of primary ovarian tumors tested. All patients had optimal cytoreductive surgery followed by adjuvant platinum based chemotherapy. Seven of the members of PDEF negative patient population were followed for survival for from 6 months to 36 months after negative PDEF determination. The remaining two negative members were not determined in sufficient time prior to the application for this patent to permit a meaningful follow-up time period. Of the negative members, one died almost immediately after negative PDEF determination, one died in about one month and another at about nine months. Two additional negative members died at 18 and 36 months respectively. Two remaining followed negative members continue to be followed and remained alive at 4 and 9 months respectively after negative PDEF determination.

Of the thirteen members that tested PDEF positive, nine were followed for survival for from 6 to 15 months. Of the nine positive members, one died at about six months after PDEF detection. The remaining members remained alive after 4, 4, 6, 7, 7, 11, 14, and 15 months respectively. By contrast, three members of the PDEF negative group had already died by nine months and by the time of application for this patent, only one had survived past eighteen months and that one died at about 36 months. It should again be noted that survival times for each member of the group run from the time of the test for PDEF for that particular member. Survival times for the group members therefore do not run concurrently.

## Claims

1. The use of PDEF specific primer to determine prognosis of ovarian cancer **characterized in that**:
a sample of ovarian tissue cells to be tested is obtained;
mRNA is isolated from the sample;
the mRNA is treated with deoxyribonuclease;
cDNA's are generated from the treated mRNA by reverse transcription polymerase chain reaction (RT-PCR) using reverse transcriptase and PDEF specific primers are used to obtain PCR amplified product; and
the amplified product is analyzed to determine PDEF expression and the expression is compared with normal expression as an indication of prognosis of ovarian cancer.

2. The use of claim 1 **characterized in that** the PDEF specific primer comprises α T12-18 primer.

3. The use of claim 1 **characterized in that** the reverse transcriptase is M-MLV reverse transcriptase.

4. The use of claim 1 **characterized in that** the amplified product is analyzed by gel chromatography.

5. The use of claim 4 **characterized in that** the gel is from about 1 to about 2% agarose gel.

6. The use of claim 1 **characterized in that** the amplified mRNA product is translated and the translation product is then analyzed by ELISA.

7. The use of claim 1 **characterized in that** the amplified mRNA product is translated and the translation product is then analyzed by radioimmunoassay.

8. The use of claim 1 **characterized in that** the ovarian tissue is epithelial tissue.
